# EUROPÄISCHE PATENTSCHRIFT

(11) **EP 0 622 342 B1**
(45) Veröffentlichungstag und Bekanntmachung des Hinweises auf die Patenterteilung: **12.08.1998**
(21) Anmeldenummer: 94250110.7
(22) Anmeldetag: 28.04.1994
(51) Int. Cl.: C03C 3/097, C03C 4/00

(54) **Opaleszierendes Glas**
Opalescent glass
Verre opalescent

(30) Priorität: 30.04.1993 DE 4314817
(43) Veröffentlichungstag der Anmeldung: 02.11.1994
(73) Patentinhaber: IVOCLAR AG, FL-9494 Schaan (LI)
(72) Erfinder: Rheinberger, Volker, Dr., FL-9490 Vaduz (LI); Höland, Wolfram, Prof. Dr., FL-9494 Schaan (LI); Frank, Martin, Dipl.-Ing., FL-9494 Schaan (LI)
(74) Vertreter: UEXKÜLL & STOLBERG

(56) Entgegenhaltungen:
- DE-A- 1 909 187
- US-A- 4 309 219
- DATABASE WPI Section Ch, Week 8616, Derwent Publications Ltd., London, GB; Class L, AN 86-105800 & SU-A-1 183 471 (GLASS RES. INST.) 7. Oktober 1985
- CHEMICAL ABSTRACTS, vol. 103, no. 2, 15. Juli 1985, Columbus, Ohio, US; abstract no. 10377n, Seite 261 ;
- CHEMICAL ABSTRACTS, vol. 79, no. 14, 8. Oktober 1973, Columbus, Ohio, US; abstract no. 82824q, Seite 281 ;

## Beschreibung

Die Erfindung betrifft opaleszierendes Glas und insbesondere opaleszierendes Glas, welches infolge seiner chemischen Beständigkeit und seiner Verarbeitungseigenschaften in der Dentaltechnik eingesetzt werden kann.

Als Opaleszenz wird die Eigenschaft eines Materials bezeichnet, im Auflicht bläulich-weiß und im Durchlicht rot zu erscheinen. Eine genauere Abhandlung über diesen Effekt findet sich zum Beispiel in W. Vogel: Glaschemie, Verlag für Grundstoffindustrie, Leipzig, 1971.

Zur Erzielung des Opaleszenzeffektes bei technischen Gläsern wird diesen häufig ein Trübungsmittel hinzugesetzt. Als Trübungsmittel finden vor allem Fluoride, Phosphate und Borate Anwendung.

Opalgläser sind zum Beispiel aus der DE-OS 1 496 646 bekannt. Die dort beschriebenen Gläser zeichnen sich durch einen Gehalt an kristallisiertem TiO₂ aus, welches eine eigene Phase bildet und eine Trübung des Glases hervorruft. Die Gläser werden vornehmlich als Pigmente in Lacken und Anstrichen sowie als Füllstoffe in Papieren, Kunststoffen und Chemiefasern eingesetzt.

In SU-A-1183471 wird ein Opalglas beschrieben, welches zur Verwendung in Lichtstreusystemen eingesetzt wird und 2 bis 10 Gew.% physiologisch bedenkliches PbO enthält.

In der SU-A-1146288 wird ein Ionenaustauschglas und in der SU-A-379545 wird ein glaskeramisches Email beschrieben. In beiden Schriften wird nicht offenbart, daß die beschriebenen Materialien einen Opaleszenzeffekt zeigen und eine heterogene Struktur mit kontinuierlicher Glasphase und daran verteilter diskontinuierlicher Glasphase haben. Auch wird nicht offenbart, die Materialien im Dentalbereich einzusetzen.

Aus der DE-PS 33 06 648 ist eine bioaktive Glaskeramik bekannt, in deren Mikrogefüge Apatit- und Glimmerkristallphasen vorliegen. Die Glaskeramik enthält kein TiO₂ oder ZrO₂. Sie ist insbesondere für prothetische Zwecke, nämlich als Knochenersatzmaterial, vorgesehen.

Die DE-PS 2 313 074 offenbart phosphatgetrübte Opalgläser auf der Basis von SiO₂-B₂O₃-Al₂O₃-K₂O. Nachteilig an diesen Gläsern ist, daß es bei Steigerung des Al₂O₃-Gehalts auf mehr als 5 Gew.-% zu einem mitunter vollständigen Verschwinden des Opaleffektes kommt.

Schließlich sind aus der DE-PS 1 909 187 Opalgläser bekannt, die ebenfalls frei von TiO₂ und ZrO₂ sind. Der Opaleszenzeffekt dieser Gläser scheint darauf zurückzuführen zu sein, daß nach Formung der gewünschten Glasgegenstände durch Wärmebehandlung eine in-situ Phasentrennung herbeigeführt wird.

Die Anforderungen, die an ein Dentalmaterial gestellt werden, erfüllen die bekannten Opalgläser jedoch nicht. Zwar reicht ihr Opaleszenzeffekt in vielen Fällen aus, um die Opaleszenz von natürlichen Zähnen zu imitieren, sie erfüllen jedoch andere Erfordernisse für Dentalmaterialien nicht. Zum einen enthalten die herkömmlichen Gläser häufig Komponenten, die physiologisch nicht völlig unbedenklich sind. Zum anderen ist die chemische Beständigkeit der Opalgläser in vielen Fällen für eine Anwendung im Dentalgebiet unzureichend. Schließlich weisen die bisher bekannten Opalgläser den Nachteil auf, daß sie eine zahntechnisch übliche Verarbeitung nicht ohne Verlust ihres ursprünglichen Opaleszenzeffektes überstehen.

Bei der zahntechnischen Verarbeitung wird das Opalglas zu einem Granulat mit einer Korngröße von weniger als 90 µm zerkleinert und anschließend das Granulat bei einer Temperatur von bis zu 1100°C zu einem dichten Produkt gesintert. Gegebenenfalls schließen sich noch weitere thermische Behandlungen bei bis zu 1100°C an. Nach Durchlaufen einer derartigen Verarbeitung werden herkömmliche opaleszierende Gläser entweder völlig transparent oder sie kristallisieren sehr schnell zu einem weißen opaken Material aus. In beiden Fällen ist der gewünschte Opaleszenzeffekt völlig verloren gegangen.

Der Erfindung liegt daher die Aufgabe zugrunde, ein opaleszierendes Glas, ein Verfahren zu seiner Herstellung sowie seine Verwendung zur Verfügung zu stellen, wobei das opaleszierende Glas eine hohe Opaleszenz und eine ausgezeichnete chemische Beständigkeit aufweist und darüber hinaus die vorstehend geschilderte zahntechnische Verarbeitung ohne wesentliche Einbuße des ursprünglichen Opaleszenzeffektes übersteht und damit als Dentalmaterial oder Bestandteil davon eingesetzt werden kann.

Diese Aufgabe wird überraschenderweise durch das opaleszierende Glas nach den Ansprüchen 1 bis 3 sowie das Verfahren zu seiner Herstellung nach den Ansprüchen 4 bis 7 und seine Verwendung nach den Ansprüchen 8 bis 10 gelöst.

Das erfindungsgemäße opaleszierende Glas ist dadurch gekennzeichnet, daß es eine kontinuierliche Glasphase und darin verteilt eine diskontinuierliche Glasphase aufweist und es die folgenden Komponenten enthält:

| Komponente | Gew.-% |
|---|---|
| SiO₂ | 48,0 bis 66,0 |
| B₂O₃ | 0 bis 1,0 |
| Me(III)₂O₃ | 5,8 bis 20,0 |
| Me(I)₂O | 6,0 bis 22,0 |
| Me(II)O | 2,6 bis 16,0, insbesondere 3,5 bis 16,0 |
| Me(IV)O₂ | 0,5 bis 10,0 |
| P₂O₅ | 0,5 bis 5,0 |
| CeO₂ | 0 bis 3,0 |

wobei
a) die angegebene Menge Me(III)₂O₃ aus 5,8 bis 20,0 Gew.-% Al₂O₃ und 0 bis 6,0 Gew.-% La₂O₃;
b) die angegebene Menge Me(I)₂O aus 3,0 bis 15,0 Gew.-% K₂O, 3,0 bis 12,0 Gew.-% Na₂O und 0 bis 2,5 Gew.-% Li₂O;
c) die angegebene Menge Me(II)O aus 0 bis 10,0 Gew.-% CaO, 0 bis 7,5 Gew.-% BaO, 0 bis 9,0 Gew.-% MgO, 0 bis 3,5 Gew.-% ZnO und 0 bis 8,5 Gew.-% SrO; und
d) die angegebene Menge Me(IV)O₂ aus 0 bis 5,0 Gew.-% TiO₂ und 0 bis 5,0 Gew.-% ZrO₂
   gebildet ist, und
   wobei opaleszierendes Glas ausgenommen ist, welches mindestens 2,0 Gew.-% PbO enthält.

Alle Gewichtsprozentangaben sind auf das Glas bezogen.

Es ist bevorzugt, daß das Glas 0 bis 0,9 Gew.-% B₂O₃ enthält. Ganz besonders bevorzugt sind Gläser, die die folgenden Komponenten enthalten:

| Komponente | Gew.-% |
|---|---|
| SiO₂ | 53,0 - 62,0 |
| B₂O₃ | 0 - 0,9 |
| Al₂O₃ | 10,0 - 17,0 |
| K₂O | 6,0 - 15,0 |
| Na₂O | 5,0 - 11,0 |
| CaO | 2,0 - 7,0 |
| BaO | 0 - 5,5 |
| SrO | 0 - 8,5 |
| TiO₂ | 0,2 - 2,0 |
| ZrO₂ | 0 - 3,0 |
| P₂O₅ | 2,0 - 5,0 |
| CeO₂ | 0 - 1,0 |

Das erfindungsgemäße opaleszierende Glas wird in herkömmlicher Weise und insbesondere durch Schmelzen geeigneter Ausgangsmaterialien bei einer Temperatur von 1400 bis 1650°C unter Bildung einer homogenen Glasschmelze gebildet. Sofern der Opaleszenzeffekt des auf diese Weise hergestellten Glases ausreicht, kann eine sich anschließende Wärmebehandlung entfallen. Da mittels der nachstehenden Wärmebehandlung jedoch die Intensität des Opaleszenzeffektes generell gesteigert werden kann, ist es bevorzugt eine solche durchzuführen. Bei der Wärmebehandlung wird die hergestellte Glasschmelze nach oder ohne vorherige Abkühlung auf Raumtemperatur über einen gewissen Zeitraum im Temperaturbereich von 950 bis 1100°C gehalten. Die Dauer dieser Behandlung beträgt vorzugsweise 15 Minuten bis 4 Stunden.

Anhand rasterelektronenmikroskopischer Untersuchung konnte nachgewiesen werden, daß das erfindungsgemäße Glas eine mikroheterogene Struktur besitzt, und zwar eine kontinuierliche Glasphase aufweist, in der eine diskontinuierliche Glasphase verteilt ist. Die diskontinuierliche Glasphase liegt in Form von Tropfen vor, die eine mittlere Größe von ca. 150 bis 250 nm, bezogen auf die Anzahl der Tropfen, haben. Die Untersuchungen zeigten ebenfalls, daß die Größenverteilung der Tropfen eng ist.

Erstaunlicherweise ist es möglich, das erfindungsgemäße Glas den bei einer zahntechnischen Verarbeitung vorliegenden Bedingungen auszusetzen, ohne daß es zu einer Beeinträchtigung des Opaleszenzeffektes kommt. Die zahntechnische Verarbeitung des erfindungsgemäßen Glases als Dentalmaterial sieht vor, daß aus dem Glas ein Granulat mit einer Teilchengröße von vorzugsweise weniger als 90 µm gebildet wird und dieses Granulat anschließend bei Temperaturen von bis zu 1100°C, vorzugsweise 880 bis 1070°C, zusammengesintert wird. An dieses Zusammensintern können sich noch zwei bis vier weitere thermische Behandlungen bei Temperaturen von bis zu 1100°C anschließen. Überraschenderweise übersteht das erfindungsgemäße Glas alle diese Bedingungen ohne Einbuße des Opaleszenzeffektes. Vielmehr wurde festgestellt, daß in einigen Fällen die Bedingungen der zahntechnischen Verarbeitung sogar zu einer Steigerung des Opaleszenzeffektes führten. Demnach ist das erfindungsgemäße Glas den bisher bekannten Gläsern deutlich überlegen, die unter den beschriebenen Bedingungen zahntechnischer Verarbeitung völlig transparent werden oder sehr schnell zu einem weißen opaken Material auskristallisieren, wobei es in beiden Fällen zu einem Verlust der Opaleszenz kommt.

Die hohe Opaleszenz der erfindungsgemäßen Gläser ist um so überraschender, als sie größere Mengen an Al₂O₃, nämlich 5,8 und bis zu 20,0 Gew.-%, enthalten. Bei den aus der DE-PS 2 313 074 bekannten Borosilikatgläsern führen solche Mengen an Al₂O₃ zu einer Verminderung oder einem völligen Verschwinden des Opaleszenzeffektes.

Neben einem stabilen Opaleszenzeffekt zeichnen sich die erfindungsgemäßen Gläser auch durch eine ausgezeichnete chemische Beständigkeit sowie einen für Dentalanwendungen geeigneten linearen Ausdehnungskoeffizienten aus. Die erfindungsgemäßen Gläser können daher vorteilhaft für dentale Anwendungen eingesetzt worden. Dabei können die Gläser selbst als Dentalmaterial oder als Bestandteil von oder Zusatz zu Dentalmaterialien verwendet werden. Besonders bevorzugt werden die erfindungsgemäßen Gläser zusammen mit Silikatgläsern und Silikatglaskeramiken eingesetzt. Alleine oder in Verbindung mit anderen kompatiblen Zusätzen können die erfindungsgemäßen Gläser wie beschrieben granuliert und anschließend zu dichten Dentalprodukten, wie Inlays, Onlays, Kronen oder Schichtkeramiken auf Metall, gesintert werden. Es ist auch möglich, das erfindungsgemäße opaleszierende Glas als Füllstoff für Inlays, Onlays oder solche Formkörper zu verwenden, aus denen computergefräste Zahnersatzteile hergestellt werden.

Die Erfindung wird anhand der nachstehenden Beispiele weiter erläutert.

### Beispiele

### Beispiele 1 bis 27

Durch Erschmelzen bei Temperaturen von 1400 bis 1650°C, Abkühlen auf Temperaturen von 1100°C bis Raumtemperatur und gegebenenfalls anschließende Wärmebehandlung während 15 Minuten bis 4 Stunden im Temperaturbereich von 950 bis 1100°C wurden 27 erfindungsgemäße opaleszierende Gläser dargestellt, die die in der nachstehenden Tabelle I ausgewiesenen Zusammensetzungen hatten.

Dabei sind die Gläser 5, 8, 9, 14, 15, 20 und 22 sowie die Gläser 25, 26 und 27 bevorzugt.

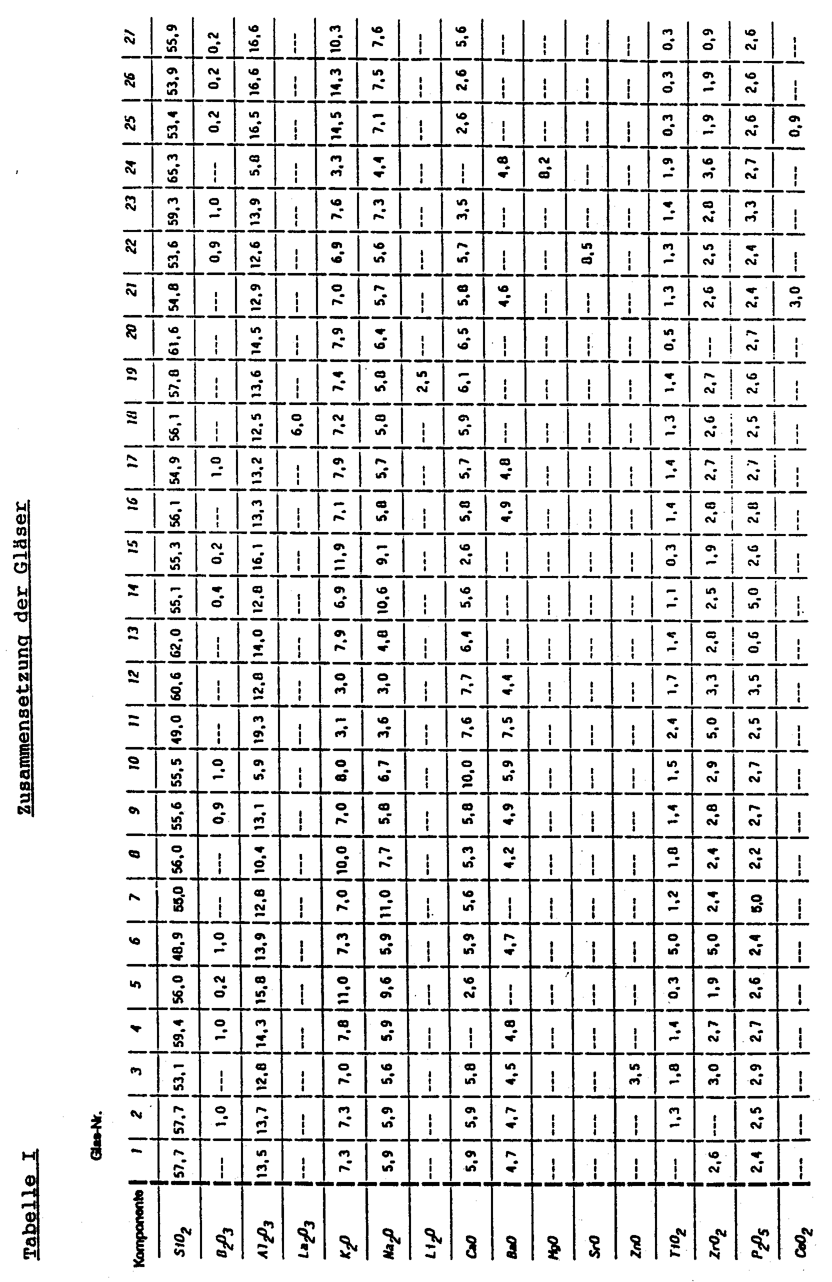

Die Eigenschaften von verschiedenen ausgewählten erfindungsgemäßen Gläsern sind in der nachstehenden Tabelle II aufgeführt.

**Tabelle II**

| **Eigenschaften ausgewählter Gläser** | | | | | |
|---|---|---|---|---|---|
| Glas-Nr. | Wärmebehandlung des Ausgangsglases nach Gießen der Schmelze [°C/h] | Sinterungstemperaturbereich zur Herstellung von Stäben für α-Messung [°C] | Linearer Ausdehnungskoeffizient α (100-500°C) [°C⁻¹] | T_{g} [°C] | T_{E} [°C] |
| 4 | keine | 1000 / 1020 | 8,1 x 10⁻⁶ | 677 | 778 |
| 5 | 950/0,5 h | 960 / 980 | 15,1 x 10⁻⁶ | 553 | 670 |
| 6 | keine | 960 / 980 | 9,1 x 10⁻⁶ | 677 | 750 |
| 8 | keine | 900 / 920 | 10,3 x 10⁻⁶ | 598 | 664 |
| 9 | 950/1 h | 1020 / 1020 | 8,5 x 10⁻⁶ | 654 | 740 |
| 9 | 1060/1 h | 1020 / 1020 | 8,4 x 10⁻⁶ | 657 | 745 |
| 13 | 1050/2 h | 960 / 980 | 8,6 x 10⁻⁶ | 672 | 748 |
| 14 | keine | 960 / 980 | 10,8 x 10⁻⁶ | 634 | 710 |
| 15 | 1000/1 h und 900/1 h | 960 / 980 | 16,8 x 10⁻⁶ | 552 | 697 |
| 16 | 1000/1 h | 1020 / 1020 | 8,7 x 10⁻⁶ | 682 | 766 |
| 18 | keine | 1030 / 1050 | 8,6 x 10⁻⁶ | 699 | 788 |
| 19 | keine | 840 / 860 | 9,5 x 10⁻⁶ | 561 | 629 |
| 20 | keine | 1000 / 1020 | 8,6 x 10⁻⁶ | 657 | 745 |
| 26 | 1040/1 h | 980 / 1000 | 18,2 x 10⁻⁶ | 580 | 770 |
| 27 | 950/0,5 h | 960 / 980 | 17,6 x 10⁻⁶ | 628 | 800 |
| T_{g} = Transformationstemperatur T_{E} = Erweichungstemperatur | | | | | |

Zur Ermittlung des linearen thermischen Ausdehnungskoeffizienten α wurden Prüfstäbe hergestellt. Dazu wurden die ausgewählten erfindungsgemäßen Gläser auf Teilchengrößen von < 90 µm granuliert und das erhaltene Glasgranulat innerhalb der in Tabelle II angegebenen Temperaturbereiche zu Stäben gesintert. Wie Tabelle II zeigt, kann der Ausdehnungskoeffizient je nach Glaszusammensetzung zwischen 8,1 x 10⁻⁶ und 18,2 x 10⁻⁶° C⁻¹, gemessen im Temperaturbereich von 100 bis 500°C, eingestellt werden. Damit ist es möglich, je nach dentaler Anwendung, zum Beispiel wenn das Glas als Zusatz für eine Aufbrennkeramik oder als Zusatz für eine Preßglaskeramik verwendet werden soll, ein Glas mit kompatiblem Ausdehnungskoeffizienten herzustellen.

In diesem Zusammenhang sind die Gläser 5, 15, 25, 26 und 27 besonders hervorzuheben. Sie zeichnen sich trotz eines sehr hohen Ausdehnungskoeffizienten durch eine ausreichende bis sehr gute Opaleszenz aus. Bei herkömmlichen Gläsern können hohe Ausdehnungskoeffizienten häufig nur unter sehr starken Einbußen beim Opaleszenzeffekt erzielt werden. Die erfindungsgemäßen Gläser vereinigen jedoch beide Eigenschaften in vorteilhafter Weise.

Weiter zeigt Tabelle II, daß die Transformationsbereiche der Gläser zwischen ca. 550 und 700°C und die Erweichungstemperaturen zwischen ca. 620 und 800°C liegen.

In der nachstehenden Tabelle III sind qualitative Werte für den Opaleszenzeffekt der erfindungsgemäßen Gläser sowie Werte für deren chemische Beständigkeit angegeben.

**Tabelle III**

| **Eigenschaften ausgewählter Gläser** | | | | |
|---|---|---|---|---|
| Glas-Nr. | Wärmebehandlung des Ausgangsglases nach Gießen der Schmelze [°C/h] | Sinterungstemperaturbereich zur Herstellung von Probeplättchen [°C] (Haltezeit 1 min) | Masseverlust nach Essigsäurebehandlung (nach ISO 6872-1984) [%] | Opaleszenz |
| 4 | keine | 1020 / 1040 | 0,0059 | gut |
| 5 | 960/1 h | 980 / 1020 | 0,0198 | ausreichend |
| 6 | keine | 980 / 1000 | 0,0143 | ausreichend |
| 8 | keine | 920 / 940 | 0,0058 | ausreichend |
| 9 | 950/1 h | 1000 / 1000 | 0,0052 | ausreichend |
| 9 | 1060/1 h | 1000 / 1020 | 0,0084 | sehr gut |
| 13 | 1050/2 h | 1000 / 1020 | 0,0153 | ausreichend |
| 14 | keine | 1000 / 1020 | 0,0092 | sehr gut |
| 15 | 950/1 h | 980 / 970 | 0,0057 | sehr gut |
| 16 | 1000/1 h | 1020 / 1000 | 0,0081 | gut |
| 18 | keine | 1050 / 1070 | 0,0060 | ausreichend |
| 19 | keine | 880 / 900 | 0,0090 | gut |
| 20 | keine | 1020 / 1040 | 0,0063 | sehr gut |
| 26 | 1040/1 h | 1010 / 1030 | 0,0348 | ausreichend |
| 27 | 950/0,5 h | 980 / 1000 | 0,0218 | gut |

Zur Feststellung der chemischen Beständigkeit der erfindungsgemäßen Gläser sowie deren Opaleszenz wurden Probeplättchen aus dem opaleszierenden Glas hergestellt, indem Granulat mit einer Teilchengröße von weniger als 90 µm innerhalb der in Tabelle III angegebenen Temperaturbereiche zu Plättchen mit einem Durchmesser von 12 mm und einer Dicke von 1 mm zusammengesintert wurde. Das Granulat wurde 1 min lang auf der Sintertemperatur gehalten.

Zur Einschätzung der chemischen Beständigkeit wurde der Masseverlust der Probeplättchen nach Behandlung mit 4 %iger Essigsäure in einer Soxhlet-Apparatur gemäß ISO 6872 - 1984 durchgeführt. Wie die in Tabelle III aufgeführten Werte zeigen, erfüllten alle Gläser die Norm von weniger als 0,05 % Masseverlust. Die angesichts eines sehr geringen Masseverlustes bevorzugten Gläser sind Nr. 4, 8, 15, 18 und 20.

Die Opaleszenzeffekte der einzelnen Gläser wurden miteinander verglichen und qualitativ bewertet. Die Ergebnisse des Bewertung finden sich in Tabelle III. Die aufgrund ihrer hohen Opaleszenz bevorzugten Gläser sind Nr. 5, 9 (mit Wärmebehandlung), 14, 15 und 20.

Um die Opaleszenz der erfindungsgemäßen Gläser auch quantitativ bestimmen zu können, wurde für drei verschiedene Gläser der sogenannte Kontrastwert (CR-Wert) gemäß British Standard Institution BSI 5612, 1978, Absatz 8.11 ermittelt. Hierzu wurde eine lichtoptische Messung im Vergleich zu einer Weiß- und Schwarz-Standardprobe an Glasprobeplättchen mit einem Durchmesser von 12 mm und einer Dicke von 1 ± 0,025 mm mit dem Gerät Minolta CR-300 durchgeführt.

Die Probeglasplättchen wurden wiederum durch Sinterung von Glasgranulat hergestellt, und der gemessene Kontrastwert sowie die qualitativ eingeschätzte Opaleszenz sind in nachstehender Tabelle IV angegeben.

**Tabelle IV**

| Glas-Nr. | Wärmebehandlung des Ausgangsglases (°C/h) | Sinterungsbedingungen zur Herstellung der Probeplättchen (°C/min) | CR-Wert | Opaleszenz qualitativ |
|---|---|---|---|---|
| 15 | 1000/0,25 | 1020/1 | 0,8559 | sehr gut |
| 16 | 1000/1 | 1020/1 | 0,7267 | gut |
| 17 | 1000/1 | 1020/1 | 0,8524 | sehr gut |

## Patentansprüche

1. Opaleszierendes Glas, **dadurch gekennzeichnet**, daß es eine kontinuierliche Glasphase und darin verteilt eine diskontinuierliche Glasphase aufweist und es die folgenden Komponenten enthält:
| Komponente | Gew.-% |
|---|---|
| SiO₂ | 48,0 bis 66,0 |
| B₂O₃ | 0 bis 1,0 |
| Me(III)₂O₃ | 5,8 bis 20,0 |
| Me(I)₂O | 6,0 bis 22,0 |
| Me(II)O | 2,6 bis 16,0, insbesondere 3,5 bis 16,0 |
| Me(IV)O₂ | 0,5 bis 10,0 |
| P₂O₅ | 0,5 bis 5,0 |
| CeO₂ | 0 bis 3,0 |
wobei
a) die angegebene Menge Me(III)₂O₃ aus 5,8 bis 20,0 Gew.-% Al₂O₃ und 0 bis 6,0 Gew.-% La₂O₃;
b) die angegebene Menge Me(I)₂O aus 3,0 bis 15,0 Gew.-% K₂O, 3,0 bis 12,0 Gew.-% Na₂O und 0 bis 2,5 Gew.-% Li₂O;
c) die angegebene Menge Me(II)O aus 0 bis 10,0 Gew.-% CaO, 0 bis 7,5 Gew.-% BaO, 0 bis 9,0 Gew.-% MgO, 0 bis 3,5 Gew.-% ZnO und 0 bis 8,5 Gew.-% SrO; und
d) die angegebene Menge Me(IV)O₂ aus 0 bis 5,0 Gew.-% TiO₂ und 0 bis 5,0 Gew.-% ZrO₂
gebildet ist, und
wobei opaleszierendes Glas ausgenommen ist, welches mindestens 2,0 Gew.-% PbO enthält.

2. Opaleszierendes Glas nach Anspruch 1, **dadurch gekennzeichnet**, daß es 0 bis 0,9 Gew.-% B₂O₃ enthält.

3. Opaleszierendes Glas nach Anspruch 1 oder 2, **dadurch gekennzeichnet**, daß es die folgenden Komponenten enthält:
| Komponente | Gew.-% |
|---|---|
| SiO₂ | 53,0 - 62,0 |
| B₂O₃ | 0 - 0,9 |
| Al₂O₃ | 10,0 - 17,0 |
| K₂O | 6,0 - 15,0 |
| Na₂O | 5,0 - 11,0 |
| CaO | 2,0 - 7,0 |
| BaO | 0 - 5,5 |
| SrO | 0 - 8,5 |
| TiO₂ | 0,2 - 2,0 |
| ZrO₂ | 0 - 3,0 |
| P₂O₅ | 2,0 - 5,0 |
| CeO₂ | 0 - 1,0. |

4. Verfahren zur Herstellung von opaleszierendem Glas nach einem der Ansprüche 1 bis 3, **dadurch gekennzeichnet**, daß ein Glas hergestellt wird, welches die Komponenten gemäß einem der Ansprüche 1 bis 3 enthält, und das hergestellte Glas gegebenenfalls einer Wärmebehandlung im Temperaturbereich von 950 bis 1100°C unterzogen wird.

5. Verfahren nach Anspruch 4, **dadurch gekennzeichnet**, daß die Wärmebehandlung 15 Minuten bis 4 Stunden lang durchgeführt wird.

6. Verfahren nach Anspruch 4 oder 5, **dadurch gekennzeichnet**, daß das hergestellte Glas granuliert und das Granulat bei Temperaturen von bis zu 1100°C zusammengesintert wird.

7. Verfahren nach Anspruch 6, **dadurch gekennzeichnet**, daß das Zusammensintern durch Erwärmen des Granulats auf 880 bis 1070°C bewirkt wird.

8. Verwendung des opaleszierenden Glases nach einem der Ansprüche 1 bis 3 als Dentalmaterial oder als Zusatz zu oder Bestandteil von Dentalmaterial.

9. Verwendung nach Anspruch 8, wobei das opaleszierende Glas als Zusatz zu Silikatgläsern oder Silikatglaskeramiken eingesetzt wird.

10. Verwendung nach Anspruch 8 oder 9, wobei das opaleszierende Glas Bestandteil von Inlays, Onlays, Brücken oder Kronen ist.

## Claims

1. Opalescent glass, characterized in that it has a continuous glass phase and, distributed therein, a discontinuous glass phase and that it contains the following components:
| Components | % by weight |
|---|---|
| SiO₂ | 48.0 to 66.0 |
| B₂O₃ | 0 to 1.0 |
| Me(III)₂O₃ | 5.8 to 20.0 |
| Me(I)₂O | 6.0 to 22.0 |
| Me(II)O | 2.6 to 16.0, in particular 3.5 to 16.0 |
| Me(IV)O₂ | 0.5 to 10.0 |
| P₂O₅ | 0.5 to 5.0 |
| CeO₂ | 0 to 3.0 |
where
a) the indicated amount of Me(III)₂O₃ is made up of from 5.8 to 20.0% by weight of Al₂O₃ and from 0 to 6.0% by weight of La₂O₃;
b) the indicated amount of Me(I)₂O is made up of from 3.0 to 15.0% by weight of K₂O, from 3.0 to 12.0% by weight of Na₂O and from 0 to 2.5% by weight of Li₂O;
c) the indicated amount of Me(II)O is made up of from 0 to 10.0% by weight of CaO, from 0 to 7.5% by weight of BaO, from 0 to 9.0% by weight of MgO, from 0 to 3.5% by weight of ZnO and from 0 to 8.5% by weight of SrO; and
d) the indicated amount of Me(IV)O₂ is made up of from 0 to 5.0% by weight of TiO₂ and from 0 to 5.0% by weight of ZrO₂,
with the exception of opalescent glass which contains at least 2.0% by weight of PbO.

2. Opalescent glass according to Claim 1, characterized in that it contains from 0 to 0.9% by weight of B₂O₃.

3. Opalescent glass according to Claim 1 or 2, characterized in that it contains the following components:
| Components | % by weight |
|---|---|
| SiO₂ | 53.0 - 62.0 |
| B₂O₃ | 0 - 0.9 |
| Al₂O₃ | 10.0 - 17.0 |
| K₂O | 6.0 - 15.0 |
| Na₂O | 5.0 - 11.0 |
| CaO | 2.0 - 7.0 |
| BaO | 0 - 5.5 |
| SrO | 0 - 8.5 |
| TiO₂ | 0.2 - 2.0 |
| ZrO₂ | 0 - 3.0 |
| P₂O₅ | 2.0 - 5.0 |
| CeO₂ | 0 - 1.0. |

4. Process for producing opalescent glass according to any of Claims 1 to 3, characterized in that a glass containing the components according to any of Claims 1 to 3 is produced and the glass produced is, if desired, subjected to a heat treatment in the temperature range from 950 to 1100°C.

5. Process according to Claim 4, characterized in that the heat treatment is carried out for from 15 minutes to 4 hours.

6. Process according to Claim 4 or 5, characterized in that the glass produced is granulated and the granulated material is sintered at temperatures of up to 1100°C.

7. Process according to Claim 6, characterized in that the sintering is effected by heating the granulated material to from 880 to 1070°C.

8. Use of the opalescent glass according to any of Claims 1 to 3 as dental material or as additive to or constituent of dental material.

9. Use according to Claim 8, wherein the opalescent glass is used as additive to silicate glasses or silicate glass-ceramics.

10. Use according to Claim 8 or 9, wherein the opalescent glass is a constituent of inlays, onlays, bridges or crowns.

## Revendications

1. Verte opalescent caractérisé en ce qu'il présente une phase vitreuse continue et une phase vitreuse discontinue dispersée dans cette phase continue et en ce qu'il contient les composants suivants :
| Composants | % en poids |
|---|---|
| SiO₂ | 48,0 à 66,0 |
| B₂O₃ | 0 à 1,0 |
| Me(III)₂O₃ | 5,8 à 20,0 |
| Me(I)₂O | 6,0 à 22,0 |
| Me(II)O | 2,6 à 16,0, en particulier 3,5 à 16,0 |
| Me(IV)O₂ | 0,5 à 10,0 |
| P₂O₅ | 0,5 à 5,0 |
| CeO₂ | 0 à 3,0 |
dans lequel
a) la quantité indiquée de Me(III)₂O₃ est constituée de 5,8 à 20,0 % en poids de Al₂O₃ et de 0 à 6,0 % en poids de La₂O₃;
b) la quantité indiquée de Me(I)₂O est constituée de 3,0 à 15,0 % en poids de K₂O, de 3,0 à 12,0 % en poids de Na₂O et de 0 à 2,5 % en poids de Li₂O;
c) la quantité indiquée de Me(II)O est constituée de 0 à 10,0 % en poids de CaO, de 0 à 7,5 % en poids de BaO, de 0 à 9,0 % en poids de MgO, de 0 à 3,5 % en poids de ZnO et de 0 à 8,5 % en poids de SrO ; et
d) la quantité indiquée de Me(IV)O₂ est constituée de 0 à 5,0 % en poids de TiO₂ et de 0 à 5,0 % en poids de ZrO₂
et où on exclut un verte opalescent, qui contient au moins 2,0 % en poids de PbO.

2. Verte opalescent selon la revendication 1, caractérisé en ce qu'il contient de 0 à 0,9 % en poids de B₂O₃.

3. Verte opalescent selon la revendication 1 ou 2, caractérisé en ce qu'il contient les composants suivants :
| Composants | % en poids |
|---|---|
| SiO₂ | 53,0 - 62,0 |
| B₂O₃ | 0 - 0,9 |
| Al₂O₃ | 10,0 - 17,0 |
| K₂O | 6,0 - 15,0 |
| Na₂O | 5,0 - 11,0 |
| CaO | 2,0 - 7,0 |
| BaO | 0 - 5,5 |
| SrO | 0 - 8,5 |
| TiO₂ | 0,2 - 2,0 |
| ZrO₂ | 0 - 3,0 |
| P₂O₅ | 2 - 5,0 |
| CeO₂ | 0 - 1,0 |

4. Procédé pour la préparation d'un verte opalescent selon l'une des revendications 1 à 3, caractérisé en ce qu'on prépare un verte, qui contient les composants selon l'une des revendications 1 à 3 et en ce que le verte obtenu subit éventuellement un traitement thermique dans une gamme de température comprise entre 950 et 1100°C.

5. Procédé selon la revendication 4, caractérisé en ce que le traitement thermique est effectué pendant 15 minutes à 4 heures.

6. Procédé selon la revendication 4 ou 5, caractérisé en ce que le verte préparé est granulé et en ce que le granulé est aggloméré par frittage à des températures allant jusqu'à 1100°C.

7. Procédé selon la revendication 6, caractérisé en ce que le frittage est provoqué par chauffage du granulé à une température comprise entre 880 et 1070°C.

8. Utilisation du verte opalescent selon l'une des revendications 1 à 3 comme matériau dentaire ou comme additif ou constituant d'un matériau dentaire.

9. Utilisation selon la revendication 8, dans laquelle le verte opalescent est utilisé comme additif pour des vertes au silicate ou des vitrocéramiques au silicate.

10. Utilisation selon la revendication 8 ou 9, dans laquelle le verte opalescent est un constituant d'inlays, d'onlays, de bridges ou de couronnes.
